(19) Europäisches Patentamt
European Patent Office
Office européen des brevets

(11) Numéro de publication: 0 009 445
B1

(12) # FASCICULE DE BREVET EUROPEEN

(45) Date de publication du fascicule du brevet: **18.05.83**

(21) Numéro de dépôt: **79400646.0**

(22) Date de dépôt: **14.09.79**

(51) Int. Cl.³: **C 07 D 471/04,**
C 07 D 209/88
//(C07D471/04, 221/00,
209/00)

(54) **Procédé de préparation d'hydroxy-9 ellipticine et de dérivés de celle-ci.**

(30) Priorité: **15.09.78 US 942793**

(43) Date de publication de la demande:
**02.04.80 Bulletin 80/7**

(45) Mention de la délivrance du brevet:
**18.05.83 Bulletin 83/20**

(84) Etats contractants désignés:
**AT BE CH DE FR GB IT NL SE**

(56) Documents cités:
**FR - A - 2 308 365**
**US - A - 3 933 827**

(73) Titulaire: **ANVAR Agence Nationale de Valorisation de la Recherche**
**13, rue Madeleine Michelis**
**F-92522 Neuilly-sur-Seine (FR)**

(73) Titulaire: **SANOFI, société anonyme**
**40, Avenue George V**
**F-75008 Paris (FR)**

(72) Inventeur: **Le Pecq, Jean-Bernard**
**48 Rue des Meuniers**
**F-75012 Paris (FR)**
Inventeur: **Paoletti, Claude**
**84 Rue Michel-Ange**
**F-75016 Paris (FR)**
Inventeur: **Dat-Xuong, Nguyen**
**93 Rue Roger Salengro**
**F-92160 Antony (FR)**

(74) Mandataire: **Harlé, Robert et al,**
**c/o Cabinet Harlé & Phélip 21, rue de la Rochefoucauld**
**F-75009 Paris (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 009 445 B1

(56) Documents cités
AUSTRALIAN JOURNAL OF CHEMISTRY, vol. 20, 1967,
Melbourne (AU)
L. K. DALTON et al.: "Synthesis of the tumour-inhibitory alkaloids, ellipticine, 9-methoxyellipticine, and related pyrido (4,3-b) carbazoles". pages 2715—27

TETRAHEDRON LETTERS, no. 15, avril 1978,
Oxford (GB)
J. Y. LALLEMAND et al. "Hydroxylation de l'ellipticine chez le rat: structure des deux principaux métabolites, synthése de l'hydroxy-7 ellipticine". pages 1261—64

COMPTES RENDUS de l'ACADEMIE DES SCIENCES, tome 284, série C, 1977,
Paris (FR)
D. ROUSSELLE et al. "Nouvelle voie d'accès aux dérivés de l'ellipticine et analogues", pages 377—80

JOURNAL OF THE CHEMICAL SOCIETY, Perkin Transactions I, 14, 1977,
London (GB)
A. H. JACKSON et al. "A new approach to the synthesis of Ellipticines", pages 1698—1704

JOURNAL OF MEDICINAL CHEMISTRY, vol. 18, no. 7, 1975,
Columbus, Ohio, USA
R. W. GUTHRIE et al. "Ellipticine Derivatives", pages 755—760

S. PATAI: "The chemistry of the hydroxyl group". Part 2. 1971, Interscience publishers

SYNTHESIS 1977
Stuttgart (DE)
M. SAINSBURY "The synthesis of 6H-pyrido (4,3-b) carbazoles". pages 437—448

Procédé de préparation d'hydroxy-9 ellipticine et de dérivés de celle-ci

La présente invention concerne la préparation d'hydroxy-9 ellipticine, ainsi que de dérivés de celle-ci substitues sur les positions 6 et/ou 9, et/ou quaternisés sur l'atome d'azote en position 2. L'invention a plus particulièrement pour objet un procédé pour la préparation de composés de formule:

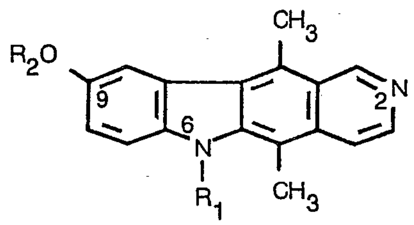

(I)

ainsi que des sels qui en dérivent par quaternisation de l'atome d'azote en 2 et qui ont pour formule:

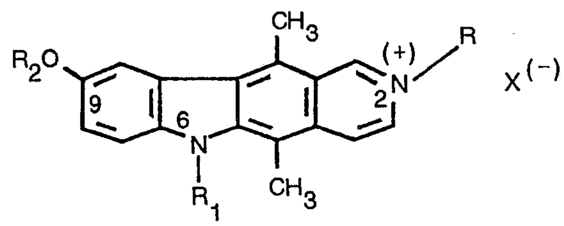

(I')

dans lesquelles $R_1$ est un atome d'hydrogène ou un groupe alkyle, $R_2$ est un atome d'hydrogène ou un groupe benzyle ou benzoyle, et R et X, lorsqu'ils sont présents, sont respectivement un groupe alkyle, éventuellement substitué par au moins un groupe hydroxy ou par un groupe amino, qui peut lui-méme étre substitué par des groupes alkyle ou faire partie d'un hétérocycle azoté, et un atome d'halogène ou un anion de quaternisation donnant un sel hydrosoluble.

On rappellera que l'hydroxy-9 ellipticine, ou hydroxy-9-diméthyl-5,11-(6H)pyrido [4,3-b] carbazole, est le composé de formule I dans lequel $R_1 = R_2 = H$.

On a déjà décrit l'hydroxy-9 ellipticine et des dérivés, notamment des sels, de celle-ci dans les brevets GB—A—1.436.080 et GB—A—1.508.388, ainsi que dans le brevet US—A—3.933.827.

Toutefois, le procédé pour la préparation de ces composés préconisé dans ces brevets GB, ainsi que dans le brevet US—A—3.933.827 repose sur une déméthylation de la méthoxy-9 ellipticine, elle-même obtenue par extraction à partir de sources naturelles ou par synthèse (voir en particulier J. W. Loder, Aust. J. Chem. 1967, *20*, pp. 2715—2727, et J. Poisson et C. Miet, Ann. Pharm. Franc. 1967, *25*, p.523). Cette déméthylation s'effectue essentiellement en présence de chlorhydrate de pyridine et à température élevée. Il est apparu que de 10 à 30% environ du produit obtenu peut être constitué par un dimère, ce qui représente une gêne et peut nécessiter une étape supplémentaire de purification.

On a maintenant trouvé de façon inattendue qu'il est possible de préparer, dans des conditions économiques nettement améliorées et avec un degré de pureté considérablement plus élevé, de l'hydroxy-9 ellipticine, ainsi que des dérivés de celle-ci substitués sur les positions 6 et/ou 9, et/ou quaternisés sur l'atome d'azote en position 2, par une voie de synthèse en plusieurs étapes qui sont associées pour constituer un procédé original procurant des résultats exceptionnels.

L'invention a donc pour objet un procédé pour la préparation d'hydroxy-9-ellipticine ou de ses dérivés de formule:

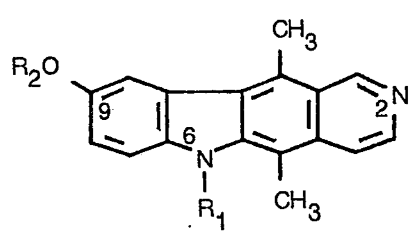

ou de leurs sels quaternisés sur l'atome d'azote de la position 2, de formule:

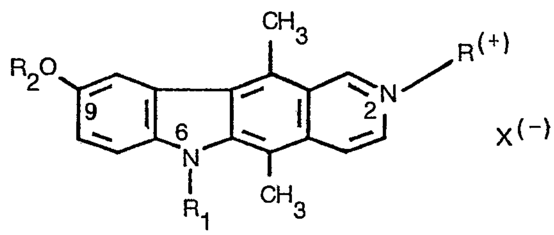

dans lesquelles $R_1$ est un atome d'hydrogène ou un groupe alkyle, $R_2$ est l'hydrogène ou un groupe benzyle ou benzoyle, R est un groupe alkyle, éventuellement substitué par au moins un groupe hydroxy ou par un groupe amino, qui peut lui-même être substitué par des groupes alkyle ou faire partie d'un hétérocycle azoté, et X est un atome d'halogène ou un anion de quaternisation donnant un sel hydrosoluble, selon lequel on met en oeuvre du méthoxy-6 dimèthyl-1,4 carbazole, obtenu par condensation d'hexane-dione- 2,5 sur du méthoxy-5 indole, caractérisé en ce que:

1 — on déméthyle le méthoxy-6 diméthyl-1,4 carbazole pour préparer l'hydroxy-6 diméthyl-1,4 carbazole,

2 — on protège le groupe hydroxy de ce dernier par benzylation ou par benzoylation de l'hydroxy-6 diméthyl-1,4 carbazole,

3 — on effectue, si on le désire, une métallation du benzyl(ou benzoyl)-oxy-6 diméthyl-1,4 carbazole ainsi préparé, et on traite le produit de cette métallation par de l'iodure d'alkyle pour préparer l'alkyl-benzyl(ou benzoyl)-oxy-6 diméthyl-1,4-carbazole,

4 — on formyle le produit de l'étape de métallation ou de l'étape précédente pour préparer le formyl-3 benzyl(ou benzoyl)-oxy-6 diméthyl-1,4 carbazole ou le formyl-3 alkyl-9 benzyl(ou benzoyl)-oxy-6 diméthyl-1,4 carbazole,

5 — on condense celui-ci sur le diméthyl-acétal de l'amino-acétaldéhyde pour préparer le (β,β-diméthoxy-éthyl-iminométhyl)-3 alkyl-9 benzyl (ou benzoyl)-oxy-6-diméthyl-1,4 carbazole, ou son homologue alkylé sur la position 9,

6 — on cyclise le produit obtenu, et si on le désire,

7 — on saponifie la benzoyl-oxy-9-ellipticine ou la benzoyl-oxy-9 alkyl-6 ellipticine ou on soumet à une hydrogénolyse la benzyloxy-9 ellipticine ou la benzyloxy-9 alkyl-6 ellipticine ainsi obtenue pour préparer l'hydroxy-9 ellipticine ou l'hydroxy-9 alkyl-6 ellipticine correspondante, et si on le désire,

8 — on traite le produit de l'étape précédente avec un halogénure d'alkyle pour obtenir l'halogénure d'hydroxy-9 alkyl-2 ellipticinium ou l'halogénure d'hydroxy-9 dialkyl-2,6 ellipticinium correspondant, et éventuellement,

9 — on convertit par échange d'ions l'halogénure d'ellipticinium substitué obtenu en un sel hydrosoluble correspondant quaternisé sur l'atome d'azote en position 2.

Pour chacune des phases opératoires ainsi définies, l'homme de l'art est à même de déterminer et de choisir après de simples essais de routine, si nécessaire, les conditions opératoires les plus appropriées; en particulier, il faut noter que l'halogénure d'alkyle utilisé pour l'alkylation sur la position 2 de la structure d'ellipticinium peut être aussi bien un halogénure d'alkyle non substitué, notamment un halogénure d'alkyle inférieur non substitué, qu'un halogénure d'alkyle dans lequel le groupe alkyle est substitué par au moins un groupe hydroxy, ou par un groupe amino, qui peut lui-même, à son tour être substitué par des groupes alkyle, notamment des groupes alkyle inférieurs, ou faire partie d'un hétérocycle azoté.

Les schémas de réaction correspondant aux étapes du procédé défini plus haut sont illustrés plus en détail ci-après, en référence au cas particulier où l'étape 3 représente une benzoylation et où l'étape 4 est effectivement réalisée. Il est clair, cependant, que les autres variantes peuvent être substituées à celle qui est détaillée ci-après, si on le désire, et sans que ces variantes aient besoin d'être détaillées à leur tour une nouvelle fois. Ces schémas de réaction donnés à titre de simple exemple illustratif sont donc les suivants:

1. Préparation du méthoxy-6 diméthyl-1,4 carbazole, par condensation de méthoxy-5 indole avec de l'hexane-dione-2,5 en milieu toluénique, avec de l'acide p-toluène sulfonique (PTSA) comme catalyseur.

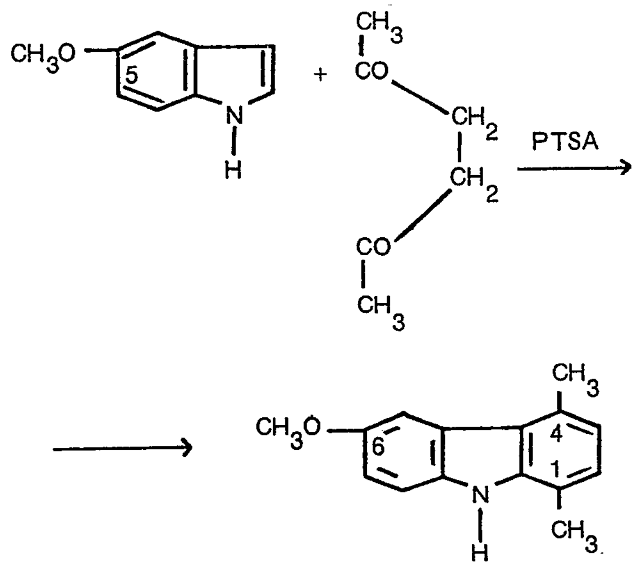


2. Préparation d'hydroxy-6 diméthyl-1,4 carbazole par déméthylation avec du chlorure de pyridinium, préparé spécialement auparavant et utilisé tant comme solvant que comme agent déméthylant.

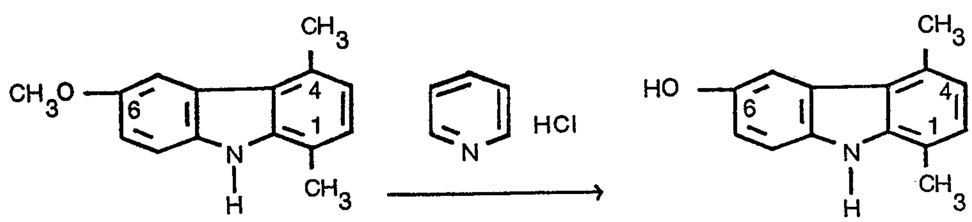


3. Protection du groupe hydroxy par préparation du benzoyl-oxy-6 diméthyl-1,4 carbazole, par réaction de l'hydroxy-6 diméthyl-1,4 carbazole, dissous dans de la pyridine ou dans l'acétone, avec du chlorure de benzoyle (fraîchement rectifié), en présence de triéthylamine.

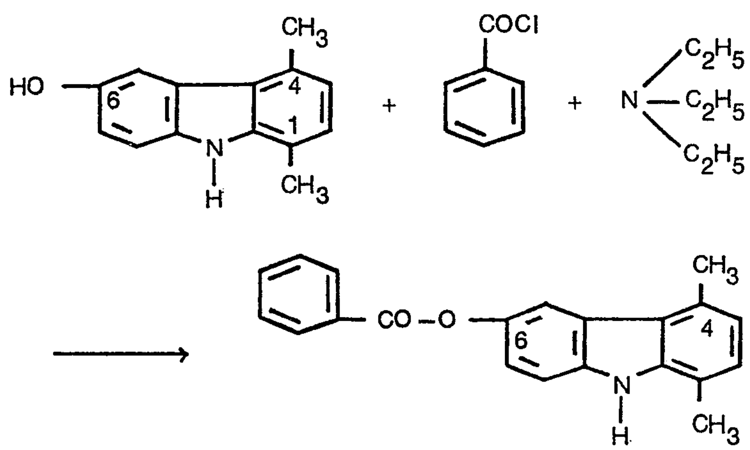

4. Préparation d'alkyl-9 benzoyl-oxy-6 diméthyl-1,4 carbazole, par métallation de benzoyl-oxy-6 diméthyl-1,4 carbazole, dissous dans le diméthylformamide, (DMF) avec NaH et traitement du dérivé contenant du sodium avec un iodure d'alkyle R—I (par exemple iodure de méthyle, iodure d'éthyle, etc. . . .).

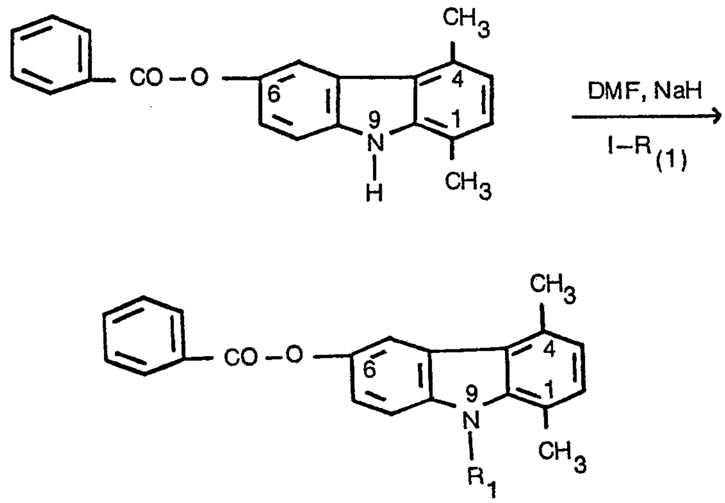


$R_1$=$CH_3$, $C_2H_5$, ou plus généralement un groupe alkyle, tel que par exemple un groupe alkyle en $C_1$—$C_3$.

5. Préparation du formyl-3 alkyl-9 benzoyl-oxy-6 diméthyl-1,4 carbazole, dans un milieu orthodichloro-benzénique, au moyen de N-méthyl formanilide en présence d'oxychlorure de phosphore.

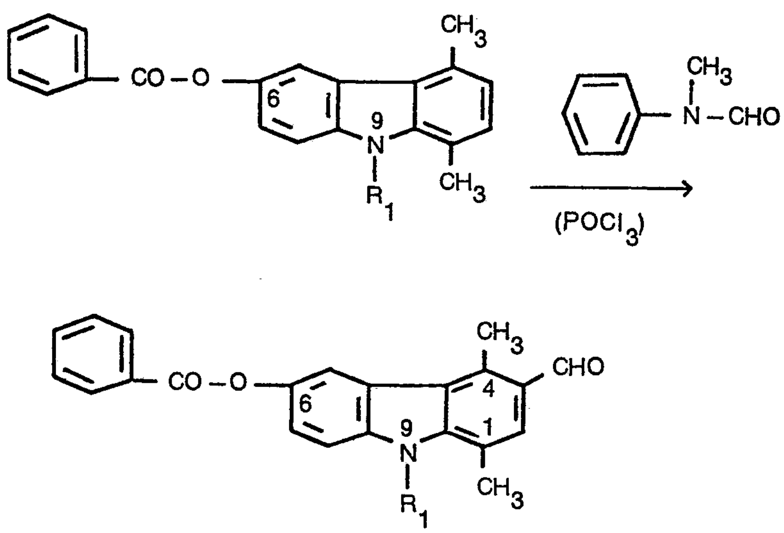


6. Préparation de (β,β-diméthoxy-éthyl-imino-méthyl)-3 alkyl-9 benzoyl-oxy-6 diméthyl-1,4 carbazole, par condensation du produit de l'étape 5 avec du diméthyl-acétal d'amino-acétaldéhyde.

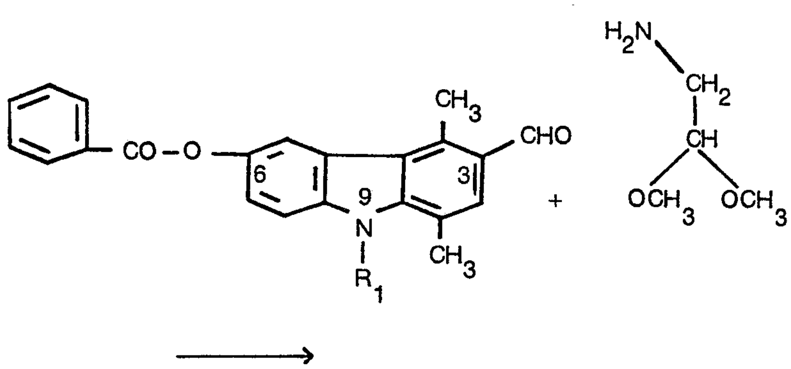

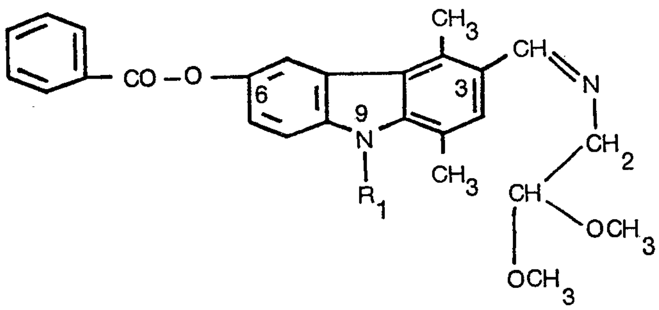

7. Préparation de benzoyl-oxy-9-alkyl-6 ellipticine(base)par cyclisation de l'azo-méthine ainsi obtenue, en présence d'un mélange de 91%. d'acide phosphonique et de pentoxyde de phosphore.

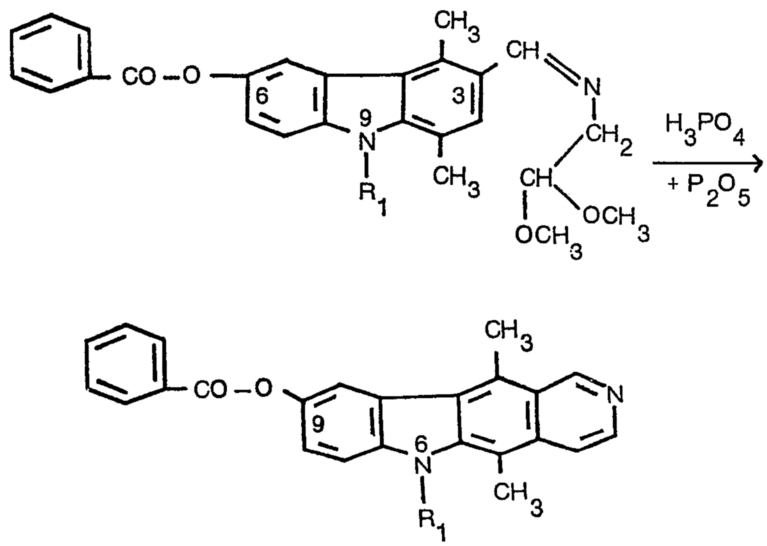


8. Préparation d'hydroxy-9 alkyl-6 ellipticine (base) par saponification de l'ester susdit, en milieu alcalin ou en milieu acide:

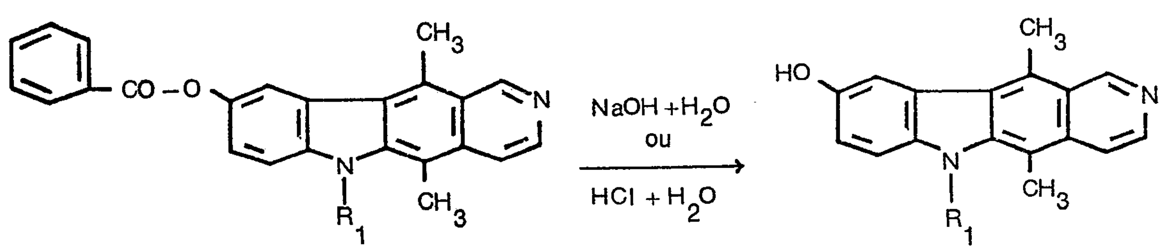


9. Préparation de l'halogénure d'hydroxy-9 dialkyl-2,6 ellipticinium(ou halogéno-alkylate d'hydroxy-9 alkyl-6 ellipticinium), par réaction du produit de l'étape 8 avec un halogénure d'alkyle, tel que par exemple l'iodure de méthyle ou l'iodure d'éthyle, dans du DMF.

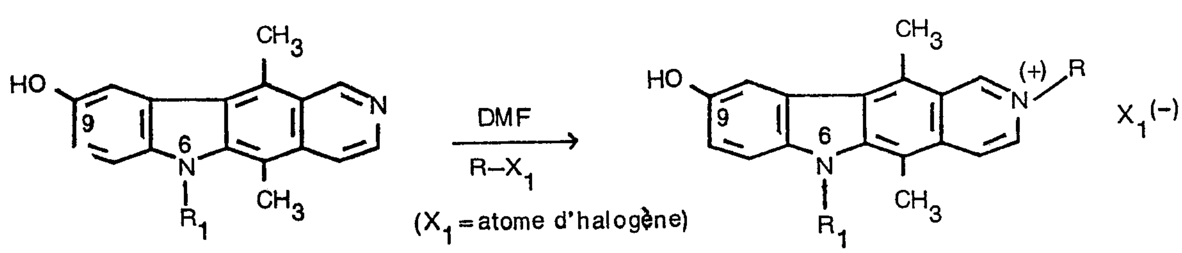


Le produit ainsi obtenu est pratiquement insoluble dans l'eau, mais soluble dans le DMF.

10. Préparation d'un sel hydrosoluble quaternisé sur l'atome d'azote en position 2 du composé de l'étape 9, par conversion en un sel hydrosoluble de cet halogénure de l'étape, de préférence par conversion en l'acétate correspondant, avantageusement par un moyen classique approprié tel qu'une résine échangeuse d'ions appropriée.

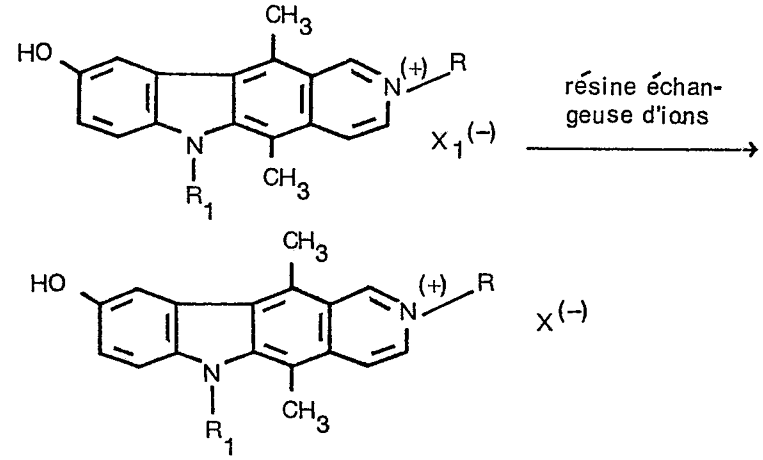


(par exemple X $=CH_3COO$).

Des exemples de résines échangeuses d'ions utilisables à cette fin sont les résines commercialisées sous les dénominations respectives Amberlite et Bio-Rad (Analytical Grade Anion Exchange Resin Bio-Rad, Ag[R] 1—X8, sous forme acétate, mise sur le marché par Bio-Rad Laboratories, Richmond, Californie, USA).

Il faut noter que ces halogénures et ces acétates, obtenus dans les étapes 9 et 10 respectivement, se décomposent avant fusion, de sorte qu'on ne peut leur attribuer un point de fusion certain. On peut par contre les identifier et les distinguer les uns des autres par leur analyse élémentaire, par leurs différences de solubilité dand le DMF ou dans l'eau, par un contrôle analytique de l'absence d'atomes d'halogène (pour la forme acétate), et de préférence même par une chromatographie en phase lipide à haute résolution. Toutes ces techniques sont bien connues de l'homme de l'art.

Ainsi qu'il ressort de la description qui en est donnée plus haut, le procédé selon l'invention est toute particulièrement approprié pour l'obtention de composés dans lesquels, en fonction des composés mis en oeuvre et/ou de l'étape du procédé que l'on considère comme l'étape finale:

—$R_1$ est H, $CH_3$ ou $C_2H_5$
—$R_2$ est H, $C_6H_5CH_2$—, $C_6H_5$—CO—.
—R est $CH_3$, $C_2H_5$,
$C_2H_4OH$, $C_3H_6OH$, $C_3H_5(OH)_2$,

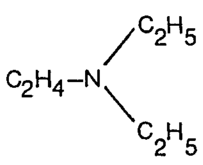
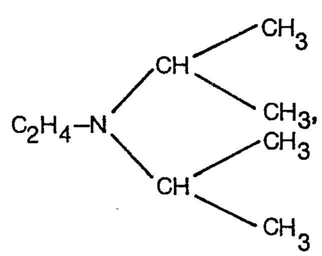
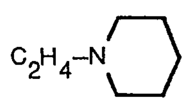

—X est un atome d'halogène, ou un groupe acyloxy, notamment le groupe $CH_3COO$.

Le procédé selon l'invention conduit à l'obtention des composés recherchés avec un très bon rendement et un haut degré de pureté. Ce dernier facteur surtout est un atoute important, puisque l'hydroxy-9 ellipticine et ses dérivés substitués sur les positions 6 et/ou 9 sont des intermédiaires pour la synthèse de sels hydrosolubles (et les dérivés quaternisés sur l'atome d'azote en position 2 sont eux-mêmes des sels hydrosolubles), qui sont en particulier utiles dans le traitement des cancers et présentent dans cette application un degré de toxicité exceptionnellement bas, comme il a été décrit dans le brevet GB—A—1.508.388, auquel il convient de se référer à ce propos.

Les composés obtenus selon l'invention sont donc essentiellement utiles pour la réalisation de compositions pharmaceutiques à activité anticancéreuse (leur efficacité dans le traitement des

leucémies et des tumeurs solides des êtres vivants ayant été établie), ou comme intermédiaires de la préparation de tels composés.

L'invention est illustrée plus en détail dans les exemples ci-après.

### Exemple 1

*Obtention d'acétate d'hydroxy-9 diéthyl-2,6 ellipticinium.*

*1. méthoxy-6 diméthyl-1,4 carbazole (produit connu et décrit dans Aust. J. Chem. 1967, 28, p. 2722).*

Dans un ballon (muni d'un agitateur magnétique, de moyens de réfrigération et d'un système d'élimination d'eau), on a versé un mélange de 117 g (0,80 mole) de méthoxy-5 indole, 300 ml d'éthanol absolu, 92 g (0,80 mole) d'hexane-dione-2,5 (fraîchement rectifiée) et 90 g d'acide p-toluène sulfonique (déshydraté azéotropiquement)et on a chauffé à reflux pendant 1 heure; on a ensuite refroidi et on a ajouté 250 ml de toluène anhydre-. On a chauffé à nouveau le mélange pour réaliser une distillation azéotropique douce, afin d'éliminer l'eau, soit approximativement 30 ml d'eau, pendant environ 2 heures.

Après refroidissement, on a ajouté 200 ml d'eau, on a séparé par décantation la couche toluénique, et on a extrait la couche aqueuse avec 100 ml de toluène.

On a recueilli les solutions toluéniques, on les a lavées deux fois avec de l'eau, on a séché sur du $CaCl_2$ anhydre, on a filtré et on a éliminé au bain-marie, sous vide, le toluène en excès.

On a recristallisé le résidu dans du cyclohexane.

Rendement: environ 78 g
cristaux fins incolores. P.F.=137°C
(point de fusion).

*2. hydroxy-6 diméthyl-1,4 carbazole.*

On a chauffé, sous doux reflux, à 180—190°C pendant 5 heures une solution de 30 g de méthoxy-6 diméthyl-1,4 carbazole dans 300 g de chlorhydrate de pyridine absolument anhydre.

Après refroidissement, on a versé le mélange sur de la glace, et on a alcalinisé avec de l'ammoniaque concentrée à un pH 6—6,5 et on a ensuite sèché, lavé plusieurs fois avec de l'eau et cristallisé à nouveau dans un mélange éthanol/eau.

Rendement: 22 g de cristaux fins incolores.
p.m. = 171°C.
$C_{14}H_{13}NO$
poids moléculaire: 211,26

| | C% | H% | N% | 0% |
|---|---|---|---|---|
| calculé | 77,84 | 5,60 | 4,32 | 12,35 |
| trouvé | 77,41 | 5,64 | 3,97—3,92 | 13 |

*3. benzoyl-oxy-6 diméthyl-1,4 carbazole*

On a dissous 21 g (0.10 mole) d'hydroxy-6 diméthyl-1,4 carbazole dans un mélange de 200 ml d'acétone anhydre et de 50 ml de triéthylamine (séchée sur des pastilles de potasse).

On a ajouté goutte à goutte 17 g (0,12 mole) de chlorure de benzoyle rectifié tout en maintenant l'agitation magnétique du mélange. La durée de la réaction est de 2 heures.

On a observé une précipitation de chlorhydrate de triéthylamine. On a éliminé par chauffage sur un bain-marie l'excès d'acétone et de triéthylamine résiduelle. On a dissous à nouveau le produit dans de l'eau et on a extrait plusieurs fois avec du $CHCl_3$. On a lavé la solution de chloroforme avec de l'eau, puis avec une solution à 5% de bicarbonate de sodium et à nouveau avec de l'eau, on a ensuite sèché sur du $CaCl_2$, on a filtré et on a éliminé le chloroforme en excès.

On a cristallisé à nouveau le produit dans une quantité minimale de $CHCl_3$.

$C_{21}H_{17}NO_2 + \frac{1}{2} H_2O$
p.m. = 315 + 9

| | C% | H% | N% | 0% |
|---|---|---|---|---|
| calculé | 77,84 | 5,60 | 4,32 | 12,35 |
| trouvé | 77,41 | 5,64 | 3,97—3,92 | 13 |

*4. éthyl-9 benzoyl-oxy-6 diméthyl-1,4 carbazole.*

Dans un appareil muni d'un piège à $CaCl_2$, on a dissous dans 120 ml de DMF (séché sur des tamis moléculaires) 16 g (0,05 mole) de benzoyl-oxy-6 diméthyl-1-1,4-carbazole. Sous agitation

magnétique, on y a ajouté 1,35 g de NaH à 100%. Après environ 1 heure il s'est formé un dérivé monosodique.

Tout en refroidissant extérieurement avec de l'eau glacée, on a ajouté goutte à goutte en 30 minutes 9 g d'iodure d'éthyle. On a laissé le mélange reposé sans agitation pendant 1 nuit. Après cela, on l'a chauffé pendant 1 heure sur un bain-marie.

On a éliminé la majeure partie du DMF sous un vide profond. Après refroidissement, on a redissous le produit dans de l'eau glacée et on l'a extrait plusieurs fois avec du $CHCl_3$.

On a lavé la solution chloroformique avec de l'eau, on a sèché sur $CaCl_2$, filtré, et on a éliminé l'excès de $CHCl_3$.

$C_{23}H_{21}NO_2$
pm = 143,4

*5. éthyl-9 benzoyl-oxy-6 formyl-3 diméthyl-1,4 carbazole.*

On a dissous dans 200 ml d'ortho-dichloro-benzène sec 41 g (0,12 mole) du produit de l'étape 4, on a ensuite ajouté 22 g (0,7 mole) de N-méthyl- formanilide fraîchement rectifiée et on a ajouté par fractions 22 g (0,14 mole) d'oxy-chlorure de phosphore.

On a chauffé pendant 3 heures le mélange total sur un bain-marie bouillant. Après refroidissement, on a versé soigneusement le produit de réaction dans une solution froide de 50 g d'acétate de sodium dans 300 ml d'eau.

On a séparé par décantation la couche benzénique et on a utilisé une extraction à la vapeur pour éliminer l'ortho-dichlor-benzène et le N-méthyl-formanilide en excès. On a dissous le résidu, en chauffant, dans du toluène et on a purifié sur du charbon.

$C_{24}H_{21}NO_3$
pm = 371,436.

*6. éthyl-9 benzoyl-oxy-6 (β,β-diméthoxy-éthyl-imino-méthyl(-3) diméthyl-1,4 carbazole.*

On a chauffé pendant 2 heures sur un bain-marie bouillant un mélange de 18,60 g (0,05 mole) du produit (aldéhyde) de l'étape 5 et 50 ml de diméthyl-acétal d'amino-acétaldéhyde. On a ensuite ajouté 100 ml de benzène anhydre et on a éliminé, par distillation azéotropique du mélange, l'eau qui s'était formée durant la réaction. On a recristallisé le résidu dans du benzène (ou du toluène).

$C_{28}H_{30}N_2O_4$
pm=458,56.

*7. benzoyl-oxy-9 éthyl-6 ellipticine (base).*

On a introduit dans une solution de 600 g d'acide ortho-phosphorique et de 20 g de pentoxyde de phosphore 22 g (0,05 mole) du produit (azométhine) de l'étape 6.

On a chauffé doucement le mélange jusqu'à 125—130°C et on l'a maintenu à cette température pendant 20 minutes. Après refroidissement ultérieur, on a versé le mélange de réaction dans 3 litres d'eau glacée, et on a effectué avec soin une neutralisation, au moyen d'ammoniaque jusqu'à un pH d'environ 7.

On a obtenu un mélange de benzoyl-oxy-9 éthyl-6 ellipticine et d'hydroxy-9 éthyl-6 ellipticine, qu'on a pu séparé par chromatographie sur colonne.

$C_{26}H_2N_2O_2$
(composé du titre)
pm=394,48.

*8. hydroxy-9 éthyl-6 ellipticine (base).*

On a chauffé à reflux doux sur un bain-marie pendant 30 minutes 10 g du mélange susdit des 2-ellipticines, dans 100 ml d'éthanol à 95% et 10 ml d'HCl concentré.

Après refroidissement et séchage, on a obtenu le composé du titre.

On a recristallisé dans de l'éthanol.

$C_{19}N_{18}N_2O$
pm=290,37.

*9. iodure d'hydroxy-9 diéthyl-2,6 ellipticinium.*
*(ou iodoéthylate d'hydroxy-9 éthyl-6 ellipticinium).*

On a dissous dans 100 ml de DMF précédemment sèché sur des tamis moléculaires 10 g d'hydroxy-9 éthyl-6 ellipticine et, après refroidissement on a ajouté encore 5 ml d'iodure d'éthyle. On a maintenu le mélange sous agitation magnétique pendant plusieurs heures, jusqu'à ce qu'ait précipité la quantité maximale d'halogénure.

On a sèché le produit et on a lavé avec de l'éther anhydre et on a ensuite recristallisé dans de l'éthanol absolu.

$C_{21}H_{23}N_3OI$
pm=446.

| | C% | H% | N% | O% | I% |
|---|---|---|---|---|---|
| calculé | 56,55 | 5.20 | 6,28 | 3,59 | 28,45 |
| trouvé | 55,77— | 5,22— | 6,11— | 3,85— | 28,96— |
| | 55,75 | 5,18 | 6,25 | 4,10 | 28,89 |

(par chromatographie en couche mince (CCM); Silicagel Merck F—254; solvant: mélange 4/1/5 de butanol acide acétique/eau; Rf ~ 0,33).

*10. acétate d'hydroxy-9 diéthyl-2,6-ellipticinium.*

On a dissous dans du DMF le produit de l'étape 9, tout en ajoutant de l'eau par fractions, de façon à former un solvant composé de DMF et d'eau par parties égales et à obtenir une concentration finale d'environ 2 à 10 mg par ml. On a ensuite dégazé soigneusement cette solution.

On a préparé comme suite une colonne de résine échangeuse d'anions appropriés:
Résine: Analytical Grade Anion Exchange Resin
Bio-Rad R AG I—X8,
forme acétate.

Quantité de résine utilisée: la capacité d'échange de cette résine était de 1,4 meq/ml de résine, ou 3,2 meq/g de poids sec de résine, c'est-à-dire dans ce cas 0,78 g de résine/g de produit. La quantité de résine respectivement utilisée était de 3 fois la quantité strictement nécessaire.

Milieu d'élution: mélange 50/50 de DMF/$H_2O$.

On a mis la résine en suspension dans ce mélange. On a ensuite alimenté la colonne et on l'a équilibrée, et on a élué le dérivé iodé (préparé comme décrit plus haut) sur cette colonne, obtenant ainsi une solution d'acétate d'hydroxy-9 diéthyl-2,6 ellipticinium. On en a éliminé complètement l'eau et partiellement le DMF. On a effectué une précipitation dans l'éther et, après filtration, le produit précipité a été préparé par filtration, sèché avec de l'éther, et sèché sous vide dans un dessiccateur. On a obtenu une poudre orange parfaitement soluble dans l'eau (rendement: 95% en poids), à partir duquel on pouvait préparer aisément une solution aqueuse contenant 4 mg par ml de cet acétate.

$C_{23}H_{26}N_2O_3$
pm=378.

## Exemple 2

*Obtention d'acétate d'hydroxy-1 éthyl-2 ellipticinium.*

En répétant le mode opératoire de l'exemple 1, mais en omettant l'étape optionnelle de métallation, à savoir l'étape 4, on a préparé successivement chacun des produits correspondant à ceux des étapes 1 à 3 et 5 à 10 de l'exemple 1, à cette différence près qu'aucun des produits obtenus dans les étapes 5 à 10 ne comportait de groupe alkyle sur l'atome d'azote en position 8 du cycle carbazole (ou en position 6 du cycle ellipticine).

En ce que concerne les produits respectivement obtenus dans les étapes 3 et 5—8 correspondantes, ils repondaient aux caractéristiques suivantes, et en outre l'analyse élémentaire a, pour chacun d'eux, permis d'établir une stricte conformité avec la formule brute présumée.

— Produit de l'étape 3: PF (point de fusion):200°C Par chromatographie en couche mince (CCM) avec élution par un mélange chlorure de méthylène-éthanol 30:1, on a obtenu une monotache, révélatrice de la pureté du produit.

— Produite de l'étape 5: PF: 264°C CCM (benzène-acétate d'éthyle 15:1):monotache.

— Produit de l'étape 6: PF: 238°C CCM (chlorure de méthylène-éthanol 20:1):monotache.

— Produit de l'étape 7: PF:245°C CCM (chlorure de méthylène-éthanol 25:5):monotache.

— Produit de l'étape 8: PF:360°C CCM (chlorure de méthylène méthanol 20:10):monotache L'hydroxy-9 ellipticine obtenue avait une masse moléculaire de 262. Son analyse était conforme à la théorie, en admettant la présence d'une demie molécule de $H_2O$ emprisonnée. D'autre part, on a pu établir qu'elle était pur à 97—98%.

Exemple 3

*Obtention d'hydroxy-9-ellipticine à partir de benzyloxy-6 diméthyl-1,4 carbazole*

On a tout d'abord préparé de l'hydroxy-6 diméthyl-1,4 carbazole en suivant le mode opératoire des étapes 1 et 2 de l'exemple 1.

On a ensuite réalisé la synthèse de l'hydroxy-9 éllipticine en suivant les enchaînements réactionnels ci-dessous, et en travaillant conformément aux modes opératoires dont le détail est donné plus loin. Sous chacune des formules des composés considérés dans les schémas réactionnels figure le poids moléculaire du composé.

Diméthyl-1,4 benzyloxy-6 carbazole

obtenu par action du chlorure de benzyle sur le diméthyl-1,4 hydroxy-6 carbazole

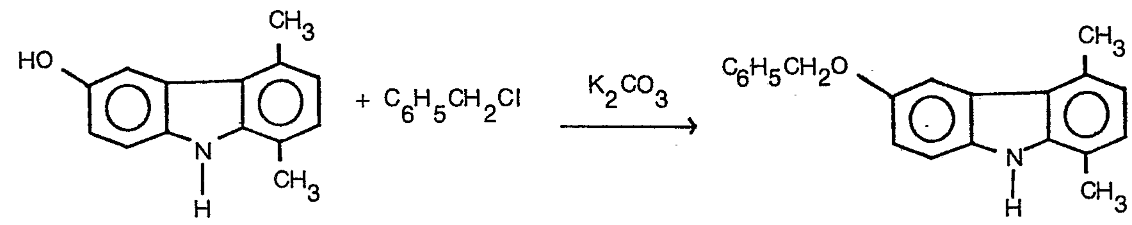


211 301

Diméthyl-1,4 formyl-3 benzyloxy-6 carbazole

obtenu par formylation du diméthyl-1,4 benzyloxy-6 carbazole.

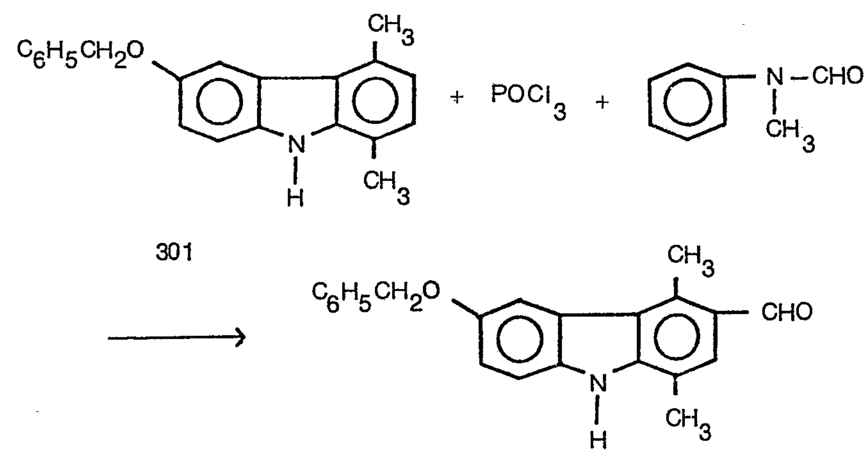


301

329

Diméthyl-1,4 ($\beta$,$\beta$-diméthoxy éthyliminométhyl)-3 benzyloxy-6 carbazole

Obtenu par action du diméthylacétal de l'aminoacétaldéhyde sur le diméthyl-1,4 formyl-3 benzyloxy-6 carbazole.

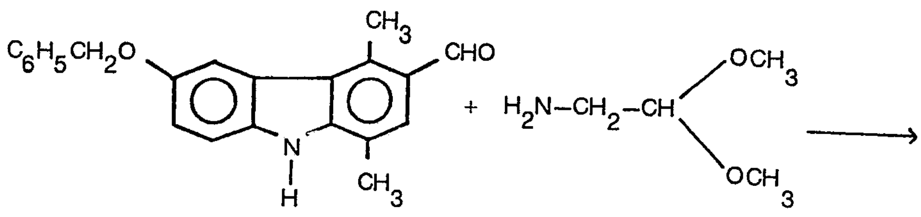

329 105

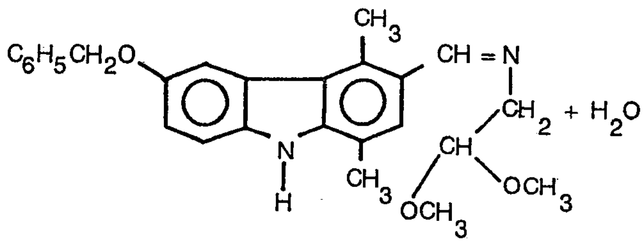

416

Benzyloxy-9 ellipticine.

Obtenu par cyclisation en milieu acide du diméthyl-1,4 ($\beta$,$\beta$-diméthoxy 2-éthyliminométhyl)-3-benzyloxy-6-carbazole.

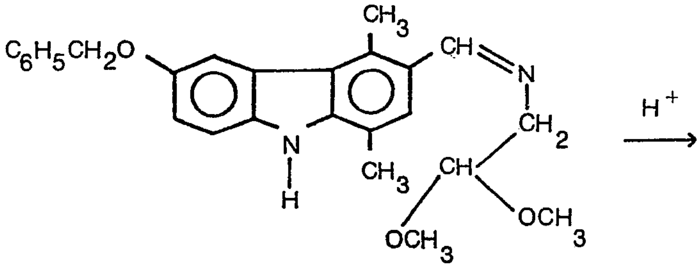


414

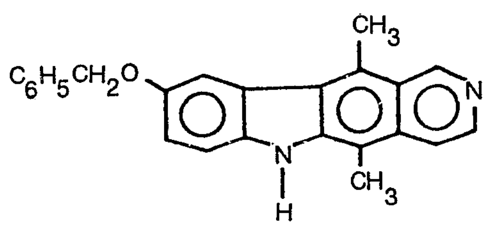

352

Hydroxy-9 ellipticine

Obtenu par hydrogénolyse de la benzyloxy-9 ellipticine.

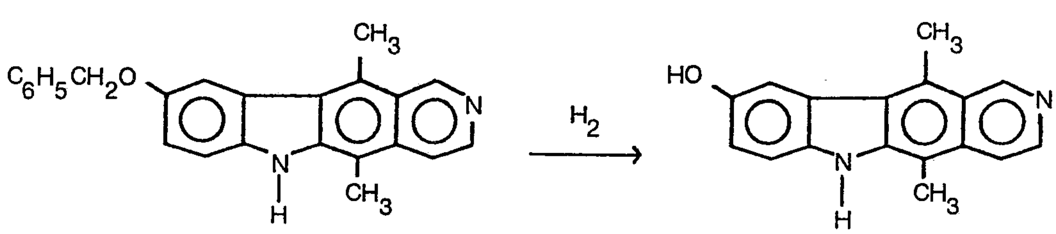


352 262

*Modes Opératoires*

Diméthyl-1,4 hydroxy-6- carbazole

Dans un ballon tricol, on a chargé 790 g (10 moles) de pyridine puis on a fait barboter par tube plongeant 112,5 g (5 moles) d'acide chlorhydrique gazeux. On a ét ête par distillation jusqu'à obtention d'un température masse de 192—195°C. On a refroidi vers 130°C et introduit 112,5 (0,5 mole) de diméthyl-1,4 méthoxy-6 carbazole. On a élevé la température à 192—195°C et maintenu cette température pendant 0,50 heure. On a hydrolysé sur 1000 g d'eau glacée. On a extrait par trois fois 300 cm3 d'éther sulfurique. On a lavé les couches éthérées réunies par deux fois 100 cm3 d'eau puis on a séché par sulfate de sodium anhydre. On a éliminé l'éther par concentration sous pression atmosphérique. On a recueilli le produit:

— Poids du produit brut: 99 g

Rendement: 94%

On a recristallisé le produit brut dans 10 volumes d'un mélange 50/50 (volume/volume) dichloroéthane-n-heptane.

— Rendement de purification: 65%

— PF du produit purifié: 167°C.

— Chromatographie couche mince:

Plaque Merck F 254

Eluant: benzène

Révélation: U.V. courts

Rf = 0,10—0,12

La déméthylation du diméthyl-1,4 méthoxy-6 carbazole, contrairement à celle de la méthoxy-9 ellipticine ne pose pas de problème de puretè pour le diméthyl-1,4 hydroxy-6- carbazole obtenu, car ce dernier est un produit stable dans les conditions opératoires retenues pour cette réaction.

Diméthyl-1,4 benzyloxy-6 carbazole

Dans un ballon tricol, on a chargé 12,6 g de diméthyl-1,4 hydroxy-6 carbazole, 100 g de N,N diméthylformamide, 16,0 g de chlorure de benzyle et 16,8 g de carbonate de potassium anhydre. On a maintenu pendant 20 h sous agitation à température ambiante. On a hydrolysé le milieu réactionnel sur 500 g d'eau. On a extrait par 2 fois 100 cm3 d'éther sulfurique et lavé les couches éthérées réunies par deux fois 50 cm3 d'eau, puis on a sèché sur sulfate de sodium anhydre. On a éliminé l'éther par concentration sous pression atmosphérique. On a repris le résidu par 50cm3 de pentane. On a essoré le précipité. On a recristallisé dans 40 cm3 d'un mélange 60/40 (volume/volume), d'heptane et de dichloroéthane.

Poids obtenu: 12,4 g    Rendement: 69%

PF = 128°C

Chromatographie couche mince: Plaque Merck F 254

Eluant: Benzène

Révelation: U.V. courts

$R_f$: 0,60.

Diméthyl-1,4 formyl-3 benzyloxy-6 carbazole

Dans un ballon tricol, on a chargé 6,02 g de diméthyl-1,4 benzyloxy-6 carbazole, 20 cm3 de dichloréthane et 3,7 g de N-méthyl formanilide, On a introduit sur ce milieu à 20°C en 0,50 h 3,7 g d'oxychlorure de phosphore. On a maintenu par un bain-marie une température masse de 50°C pendant 5 heures. On a hydrolysé le milieu réactionnel sur une solution de 12,3 g d'acétate de sodium trihydraté dans 350 g d'eau. On a filtré le précipité, on l'a lavé abondamment à l'eau, puis on l'a repris sous agitation dans 50 cm3 d'éther sulfurique. On a essoré à nouveau et séché.

Poids obtenu: 5,25 g    Rendement: 80%

PF = 193°C

Chromatographie couche mince: Plaque Merck F 254

Eluant: Benzène 50 cm3

Acétone 20 cm3

Révélation: U.V. courts

$R_f$: 0,80

Diméthyl-1,4 ($\beta,\beta$-diméthoxy-éthylimmométhyl)-3- benzyloxy-6 carbazole

Dans un ballon tricol, on a chargé 6,6 g de diméthyl-1,4 formyl-3 benzyloxy-6 carbazole, 50 g de benzène et 2,4 g de diméthylacétal de l'aminoacétaldéhyde. On a porté à reflux et éliminé l'eau réactionnelle par azéotropie, en deux heures. On a refroidi le milieu à + 10°C et essoré le précipité. On a rincé les cristaux obtenus par 50 cm3 de n-heptane et séché.

Poids obtenu: 6,6 g Rendement: 79%

PF = 130°C.

Benzyloxy-9 ellipticine

Dans un ballon tricol, on a chargé 43 g d'acide phosphorique 85% et 5,7 g d'anhydride phosphorique. On a porté sous agitation à 80—90°C puis coulé une solution de 3,7 g de diméthyl-1,4 ($\beta,\beta$-diméthoxy-éthyliminométhyl)-3 benzyloxy-6 carbazole dans 25 cm3 de benzène. On a maintenu au reflux pendant 10 minutes, On a hydrolysé le milieu réactionnel sur 100 g d'eau, puis neutralisé à pH 9 par ajout lent d'une solution de soude à 50%. On a essoré le précipité, on l'a rincé abondamment à l'eau et séché.

Poids obtenu: 3,0 g Rendement: 95%

Chromatographie couche mince: Plaque Merck F 254

Eluant: Benzène: 72 cm3

Ethanol: 18 cm3

Révélation: U.V. courts

$R_f \simeq 0,50 (C_6H_5CH_2OH)$

$\simeq 0,35$ (HO—R)

Le produit brut est composé d'un mélange de 70% de dérivé benzylé et de 30% de dérivé hydroxylé venant d'une coupure en milieu acide. Il peut être recristallisé dans 200 volumes d'acétone, pour conduire à un produit contenant moins de 5% de dérivé hydroxylé qui ne constitue pas une impureté, puis qu'il s'agit du produit préparé dans la phase suivante.

Hydroxy-9 ellipticine

Dans un Erlenmeyer on a chargé 500 cm3 d'éthanol, 0,5 g de charbon palladié à 10% de palladium, puis 5 g de benzyloxy-9 ellipticine; Après les purges à l'azote, on a effectué l'hydrogénolyse sous 150 g de pression et à température ambiante. On a éliminé le charbon palladié après absorption de la quantité théorique d'hydrogène. On a mis à sec de façon classique au rotavapor sous pression résiduelle de la trompe à eau.

Poids obtenu: 3,7 g Rendement 100%

On a purifié le produit dans 100 volumes d'éthanol, avec élimination de l'insoluble à chaud.

Rendement de purification: 85%

PF + 255°C

Titre anhydrotitrimétrique: $\gtrless$ 98%

Pourcentage de dimère $\leqslant$ 2% (chromatographie liquide).

## Revendication

Procédé pour la préparation d'hydroxy-9 ellipticine ou des ses dérivés de formule:

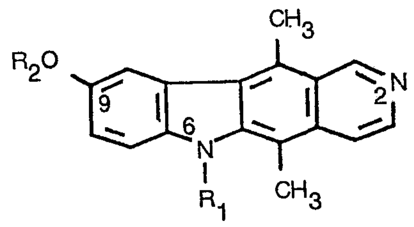

ou de leurs sels quaternisés sur l'atome d'azote de la position 2, de formule

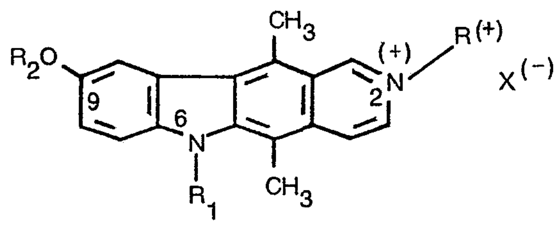

dans lesquelles $R_1$ est un atome d'hydrogène ou un groupe alkyle, $R_2$ est l'hydrogène ou un groupe benzyle ou benzoyle, R est un groupe alkyle, éventuellement substitué par au moins un groupe hydroxy ou par un groupe amino, qui peut lui-même être substitué par des groupes alkyle ou faire partie d'un hetérocycle azoté, et X est un atome d'halogène ou un anion de quaternisation donnant un sel hydrosoluble, selon lequel on met en oeuvre du méthoxy-6 diméthyl-1,4 carbazole, obtenu par condensation d'hexane-dione-2,5 sur du méthoxy-5 indole, caractérisé en ce que:

1 — on déméthyle le méthoxy-6 diméthyl-1,4 carbazole pour préparer l'hydroxy-6 diméthyl-1,4 carbazole,

2 — on protège le groupe hydroxy de ce denier par benzylation ou par benzoylation de l'hydroxy-6 diméthyl-1,4 carbazole,

3 — on effectue, si on le désire, une métallation du benzyl (ou benzoyl)-oxy-6 diméthyl-1,4 carbazole ainsi préparé, et on traite le produit de cette métallation par de l'iodure d'alkyle pour préparer l'alkyl-9 benzyl(ou benzoyl)-oxy-6 diméthyl-1,4-carbazole,

4 — on formyle le produit de l'étape de métallation ou de l'étape précédente pour préparer le formyl-3 benzyl(ou benzoyl)-oxy-6 diméthyl-1,4 carbazole ou le formyl-3 alkyl-9 benzyl (ou benzoyl)-oxy-6 diméthyl-1,4 carbazole,

5 — on condense celui-ci sur le diméthyl-acétal de l'amino-acétaldéhyde pour préparer le ($\beta$,$\beta$-diméthoxy-éthyl-iminométhyl)-3 alkyl-9 benzyl (ou benzoyl)-oxy-6 diméthyl-1,4 carbazole, ou son homologue alkylé sur la position 9,

6 — on cyclise le produit obtenu, et si on le désire

7 — on saponifie la benzoyl-oxy-9 ellipticine ou la benzoyl-oxy-9 alkyl-6 ellipticine ou on soumet à une hydrogénolyse la benzyloxy-9 ellipticine ou la benzyloxy-9 alkyl-6 ellipticine ainsi obtenue pour préparer l'hydroxy-9 ellipticine ou l'hydroxy-9 alkyl-6 ellipticine correspondante, et si on le désire,

8 — on traite le produit de l'étape précédente avec un halogénure d'alkyle pour obtenir l'halogénure d'hydroxy-9 alkyl-2 ellipticinium ou l'halogénure d'hydroxy-9 dialkyl-2,6 ellipticinium correspondant, et éventuellement,

9 — on convertit par échange d'ions l'halogénure d'ellipticinium substitué obtenu en un sel hydrosoluble correspondant quaternisé sur l'atome d'azote en position 2.

## Claim

Process for preparing 9-hydroxy ellipticine or its derivatives having the formula:

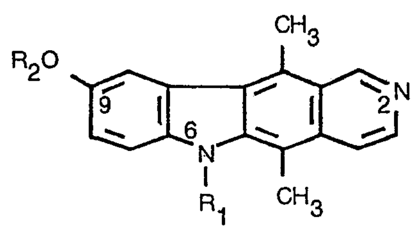

or their salts quaternized on the nitrogen atom at position 2, during the formula

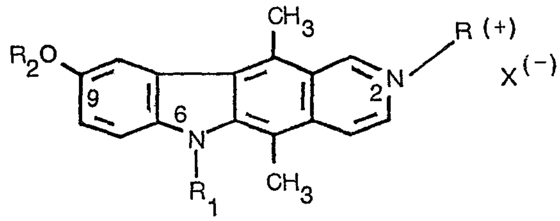

in which R, is a hydrogen atom or an alkyl group, $R_2$ is hydrogen or a benzyl or benzoyl group, R is an alkyl group, which may be substituted by at least one hydroxy group or by an amino group, which may be itself substituted by alkyl groups or be itself part of a nitrogen containing heterocycle, and X is a halogen atom or a quaternization anion giving a hydrosoluble salt, wherein there is employed 6-methoxy 1,4-dimethyl carbazole obtained by condensation of hexane-2,5-dione on 5-methoxy indole, said process comprising:

1 — demethylating the 6-methoxy 1,4-dimethyl carbazole so as to prepare 6-hydroxy 1,4-dimethyl carbazole,

2 — protecting the hydroxy group from the latter by benzylation or benzoylation of the 6-hydroxy 1,4-dimethyl-carbazole,

3 — effecting, if desired, a metallation of the 6-benzyl (or benzoyl)-oxy 1,4-dimethyl carbazole thus prepared, and treating the product of said metallation with alkyl iodide so as to prepare 9-alkyl 6-benzyl (or benzoyl)-oxy 1,4-dimethyl carbazole,

4 — formylating the product of the metallation step or of the preceding step so as to prepare the 3-formyl 6-benzyl (or benzyol)-oxy 1,4-dimethyl carbazole or 3-formyl 9-alkyl 6-benzyl (or benzoyl)-oxy 1,4-dimethyl carbazole,

5 — condensing the latter on the dimethyl-acetal of amino-acetaldehyde so as to prepare 3-($\beta$,$\beta$-dimethoxyethyl-iminomethyl)-9-alkyl 6-benzyl(or benzoyl)-oxy 1,4-dimethyl carbazole or the similar compound alkylated at position 9,

6 — cyclizing the product obtained and, if desired,

7 — saponifying the 9-benzyloxy ellipticine or the 9-benzoyloxy 6-alkyl ellipticine or subjecting to a hydrogenolysis the 9-benzyloxy ellipticine or the 9-benzyloxy 6-alkyl ellipticine thus obtained so as to prepare the corresponding 9-hydroxy ellipticine or 9-hydroxy 6-alkylellipticine, and, if desired,

8 — treating the product of the preceding step with an alkyl halide so as to obtain the corresponding halide of 9-hydroxy 2-alkyl ellipticinium or the halide of 9-hydroxy 2,6-dialkyl ellipticinium, and optionally,

9 — converting by an ion exchange the substituted halide of ellipticinium obtained into a corresponding hydrosoluble salt quaternized on the nitrogen atom at position 2.

**Patentanspruch**

Verfahren zur Herstellung von 9-Hydroxyellipticin oder seiner Derivate der Formel:

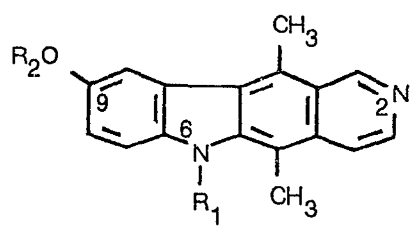

oder ihrer am Stickstoffatom in der 2-Stellung quaternisierten Salze der Formel

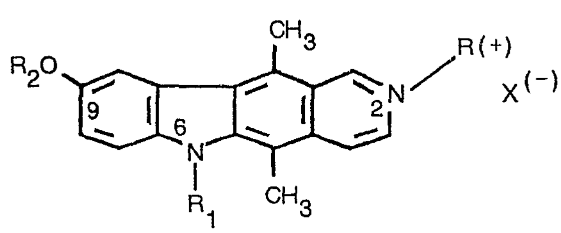

in denen $R_1$ ein Wasserstoffatom oder eine Alkylgruppe ist, $R_2$ Wasserstoff oder eine Benzyl- oder Benzoylgruppe ist, R eine Alkylgruppe ist, die gegebenenfalls durch mindestens eine Hydroxygruppe oder durch eine Aminogruppe, die ihrerseits durch Alkylgruppen substituiert oder Teil eines Stickstoffheterozyklus sein kann, substituiert ist, und X ein Halogenatom oder ein Quaternisierungsanion ist, das ein wasserlösliches Salz ergibt, in dem man 6-Methoxy-1,4-dimethylcarbazol einsetzt, das durch Kondensation von Hexan-2,5-dion und 5-Methoxyindol erhalten worden ist, dadurch gekennzeichnet, daß man

1 — 6-Methoxy-1,4-dimethylcarbazol demthyliert, um 6-Hydroxy-1,4-dimethylcarbazol herzustellen,

2 — die Hydroxygruppe des letzteren durch Benzylierung oder Benzoylierung von 6-Hydroxy-1,4-dimethylcarbazol schützt,

3 — gegebenenfalls eine Metallisierung des so hergestellten 6-Benzyl-(oder -Benzoyl)-oxy-1,4-dimethylcarbazols durchführt und das Produkt dieser Metallisierung mit Alkyljodid behandelt, um 9-Alkyl-6-benzyl-(oder benzoyl)-oxy-1,4-dimethylcarbazol herzustellen,

4 — das Produkt der Metallisierungsstufe oder der vorangehenden Stufe formyliert, um 3-Formyl-6-benzyl-(oder-benzoyl)-oxy-1,4-dimethylcarbazol oder 3-Formyl-9-alkyl-6-benzyl-(oder -benzoyl)-oxy-1,4-dimethylcarbazol herzustellen,

5 — dieses mit Aminoacetaldehyd-dimethylacetal kondensiert, um 3-($\beta$,$\beta$-Dimethoxyethyliminomethyl)-9-alkyl-6-benzyl-(oder -benzoyl)-oxy-1,4-dimethylcarbazol oder sein in der 9-Stellung alkyliertes Homolog herzustellen,

6 — das erhaltene Produkt zyklisiert und gegebenenfalls

7 — das 9-Benzoyloxyellipticin oder 9-Benzoyloxy-6-alkylellipticin verseift oder das so erhaltene 9-Benzyloxyellipticin oder 9-Benzyloxy-6-alkylellipticin hydriert, um das entsprechende 9-Hydroxyellipticin oder 9-Hydroxy-6-alkylellipticin herzustellen, und gegebenenfalls

8 — das Produkt der vorangehenden Stufe mit einem Alkylhalogenid behandelt, um das entsprechende 9-Hydroxy-2-alkylellipticiniumhalogenid oder 9-Hydroxy-2,6-dialkylellipticiniumhalogenid zu erhalten, und gegebenenfalls,

9 — das erhaltene substituierte Ellipticiniumhalogenid durch Ionenaustausch in ein entsprechendes wasserlösliches Salz überführt, das am Stickstoffatom in der 2-Stellung quaternisiert ist.